# EUROPEAN PATENT APPLICATION

(11) **EP 4 406 949 A1**
(43) Date of publication of application: **31.07.2024**
(21) Application number: 21957367.2
(22) Date of filing: 20.12.2021
(51) Int. Cl.: C07D 405/06

(54) **METHOD FOR PREPARING LIFITEGRAST AND INTERMEDIATE COMPOUND OF LIFITEGRAST**

(30) Priority: 18.09.2021 CN 202111110390
(71) Applicant: The First Affiliated Hospital, Zhejiang University School of Medicine, Hangzhou, Zhejiang 310003 (CN); Zhejiang Ausun Pharmaceutical Co., Ltd., Taizhou, Zhejiang 317016 (CN)
(72) Inventor: WANG, Xiawei, Hangzhou, Zhejiang 310003 (CN); ZHENG, Zhiguo, Linhai, Zhejiang 317016 (CN)
(74) Representative: Vossius & Partner Patentanwälte Rechtsanwälte mbB
(86) International application number: PCT/CN2021/139695
(87) International publication number: WO 2023/040103

(57) **Abstract**

The invention relates to a preparation method of lifitegrast and intermediate compounds thereof. In particular, the invention relates to a synthesis method of lifitegrast, a key intermediate for synthesizing lifitegrast and solvates thereof, and a method for preparing lifitegrast by hydrolyzing the key intermediate or solvates thereof under alkaline condition.

## Description

### Technical Field

The present invention relates to the field of synthesis of organic compounds. In particular, the invention relates to a synthetic method of lifitegrast, a key intermediate for synthesizing lifitegrast and a preparation method thereof.

### Background

Lifitegrast, a novel small molecule integrin inhibitor developed by Shire pharmaceuticals, antagonizes lymphocyte-associated antigens and blocks their interaction with their cognate ligands, intercellular adhesion molecules, and interferes with the overexpression of corneal and conjunctival tissues causing dry eye disease. It was approved for sale in the United States by the U.S. Food and Drug Administration (FDA) on July 11, 2016.

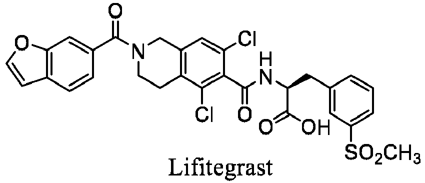

Conventional preparation methods of lifitegrast include hydrolysis or hydrogenation of the ester group of its precursor. As shown in Scheme 1, WO2009139817A2, WO2011050175A1 and WO2019096996A1 disclose that the compound of formula II is hydrogenated under the catalytic action of noble metal palladium carbon to obtain lifitegrast. However, this route has noble metal residue and the production cost is high.

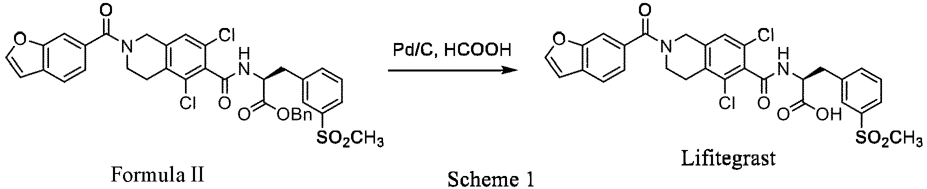

As shown in Scheme 2, WO2019097547A1 utilizes alkaline hydrolysis of the compound of formula III to obtain lifitegrast. This process seems simple, but in actual production, the chiral configuration of chiral amino acid derivatives is prone to racemization under strong alkaline conditions and produces impurity A, which is very unfavorable for the purification of lifitegrast.

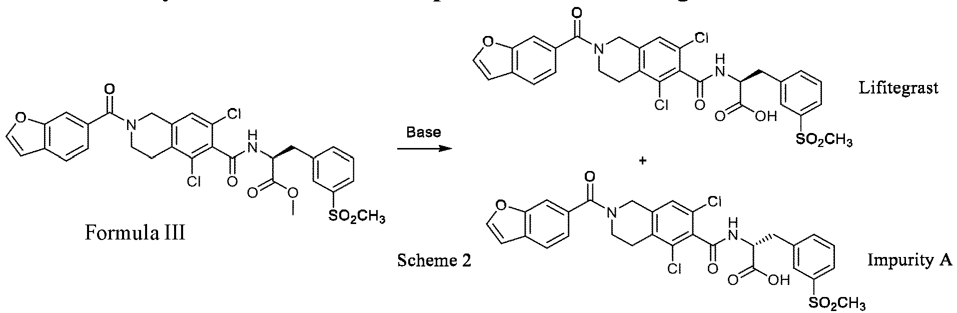

In view of the shortcomings of the existing methods, the inventors unexpectedly discovered a method suitable for industrial production of lifitegrast, which not only can effectively inhibit the generation of impurity A, but is also simple to operate and can significantly reduce production costs. Moreover, the inventors have also obtained a new crystalline form of solvate of the compound of formula III, which can be used to prepare lifitegrast with a product purity of higher than 99.9%, with single impurity less than 0.1%, even equal to zero, and the preparation method of said crystalline form is simple, thus reducing costs and solving the shortcomings of the existing methods.

### Disclosure of Invention

In the present application, the following terms have the meanings as described below:
"DMF" means N,N-dimethylformamide.
"DMA" means N,N-dimethylformamide.
"NMP" means N-methylpyrrolidone.
"DMSO" means dimethyl sulfoxide.
"DIPEA" means N,N-diisopropylethylamine.
"THF" means tetrahydrofuran.

The term "alkyl" means a straight or branched chain saturated hydrocarbon group consisting of carbon and hydrogen atoms. Preferably, the alkyl group has 1-10, such as 1 to 6, 1 to 5, 1 to 4, 1 to 3, or 1 to 2 carbon atoms. The terms "C₁₋₁₀ alkyl" and "C₁₋₆ alkyl" refer to straight or branched chain saturated hydrocarbon groups having 1 to 10 and 1 to 6 carbon atoms, respectively. Examples of alkyl groups include methyl, ethyl, n-propyl, isopropyl, n-butyl, isobutyl, tert-butyl, n-pentyl, isopentyl, neopentyl, n-hexyl, 2,2-dimethylbutyl, 2-methylpentyl, 2,3-dimethylbutyl, 3-methylpentane, cyclohexyl, n-heptyl, or n-octyl.

The term "alkoxy" means the group R'-O-, wherein R' is an alkyl as described above. "C₁₋₆ alkoxy" represents the group R'-O-, where R' is C₁₋₆ alkyl as described above.

The term "halogen" means fluorine, chlorine, bromine or iodine.

The term "haloalkyl" means an alkyl group as described above wherein one or more (e.g., 1, 2, 3, 4, or 5) hydrogen atoms are replaced by a halogen selected from the group consisting of fluorine, chlorine, bromine, and iodine. Preferably, haloalkyl is "C₁₋₁₀ haloalkyl" or "C₁₋₆ haloalkyl". Examples thereof include, for example, -CH₂F, -CHF₂, -CF₃, -CCl₃, -C₂F₅, -C₂Cl₅, -CH₂CF₃, -CH₂Cl, -CH₂CH₂CF₃ or -CF(CF₃)₂.

The term "haloalkoxy" means an alkoxy group as described above wherein one or more (e.g., 1, 2, 3, 4, or 5) hydrogen atoms are replaced by a halogen selected from the group consisting of fluorine, chlorine, bromine, and iodine. Preferably, haloalkyl is "C₁₋₁₀ haloalkoxy" or "C₁₋₆ haloalkoxy". Examples thereof include, for example, -O-CH₂F, -O-CHF₂, -O-CF₃, -O-CH₂CF₃, -O-CH₂Cl, or -O-CH₂CH₂CF₃.

The term "cycloalkyl" means a monocyclic, fused polycyclic, bridged polycyclic, or spiro non-aromatic saturated monovalent hydrocarbon ring structure having the number of ring atoms specified. The cycloalkyl group can have 3 to 12 carbon atoms (i.e., C₃-C₁₂ cycloalkyl), for example, 3 to 10, 3 to 8, 3 to 7, 3 to 6, or 5 to 6 carbon atoms. Examples of cycloalkyl groups include, but are not limited to, cyclopropyl, cyclobutyl, cyclopentyl, cyclohexyl, cycloheptyl, or cyclooctyl.

The term "aryl" means a monovalent aromatic hydrocarbon radical derived by the removal of one hydrogen atom from a single carbon atom in an aromatic ring system. Specifically, aryl means a monocyclic or fused polycyclic aromatic ring structure having the specified number of ring atoms. For example, aryl includes groups containing 6 to 14, e.g. 6 to 10, preferably 6 ring atoms. Examples of aryl groups include C₆₋₁₄ aryl or C₆₋₁₀ aryl, such as phenyl or naphthyl, preferably phenyl.

The term "aralkyl" means an alkyl group as described above substituted with an aryl group as described above, e.g., C₆-C₁₀ aryl-C₁₋₆ alkyl, e.g., benzyl.

The term "optionally substituted" means that the group may be unsubstituted or substituted with one or more (e.g., 1, 2, 3, 4 or 5 or more) substituents, which may be the same or different.

"Optionally substituted cycloalkyl" means a cycloalkyl group, e.g., C₃₋₈ cycloalkyl, optionally substituted with one, two, or three of the same or different substituents selected from C₁₋₆ alkyl, C₁₋₆ alkoxy, halogen, C₁₋₆ haloalkyl, and C₁₋₆ haloalkoxy, e.g., C₃₋₈ cycloalkyl substituted with one, two, or three substituents selected from halogen, methyl, ethyl, n-propyl, isopropyl, n-butyl, isobutyl, t-butyl, methoxy, and ethoxy.

"Optionally substituted aryl" means an aryl group optionally substituted with one, two or three of the same or different substituents selected from C₁₋₆ alkyl, C₁₋₆ alkoxy, halogen, C₁₋₆ haloalkyl and C₁₋₆ haloalkoxy, e.g., C₆-C₁₀ aryl, e.g., phenyl substituted with one, two or three substituents selected from halogen, methyl, ethyl, n-propyl, isopropyl, n-butyl, isobutyl, tert-butyl, methoxy and ethoxy.

"Optionally substituted aralkyl" means an aralkyl group optionally substituted with one, two or three of the same or different substituents selected from C₁₋₆ alkyl, C₁₋₆ alkoxy, halogen, C₁₋₆ haloalkyl and C₁₋₆ haloalkoxy, e.g., C₆-C₁₀ aryl-C₁₋₆ alkyl, e.g., benzyl substituted with one, two or three substituents selected from halogen, methyl, ethyl, n-propyl, isopropyl, n-butyl, isobutyl, t-butyl, methoxy and ethoxy.

In a first aspect, the present invention provides a method for preparing lifitegrast, which comprises:
hydrolyzing the compound of formula I or a salt or solvate thereof under alkaline condition to obtain lifitegrast, 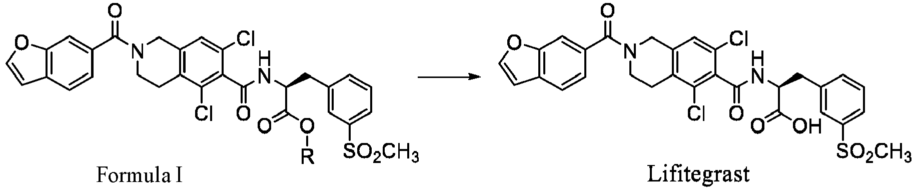
wherein R is selected from alkyl, haloalkyl, optionally substituted cycloalkyl, optionally substituted aryl or optionally substituted aralkyl, preferably C₁₋₁₀ alkyl, C₆₋₁₀ aryl, or C₆₋₁₀ aryl-C₁₋₁₀ alkyl, for example C₁₋₆ alkyl or C₆₋₁₀ aryl-C₁₋₆ alkyl, particularly preferably methyl, ethyl, n-propyl, isopropyl, n-butyl, isobutyl, tert-butyl or benzyl, and
the alkaline condition is that the hydrolysis is carried out in the presence of both an inorganic base and an organic base.

In a preferred embodiment, the inorganic base is selected from the group consisting of hydroxides, carbonates, bicarbonates, hydrides of alkali or alkaline earth metals or any combination thereof, for example, any one or a combination of any two or more of lithium hydroxide, sodium hydroxide, potassium hydroxide, calcium hydroxide, Ba(OH)₂, sodium carbonate, potassium carbonate, cesium carbonate, sodium bicarbonate, potassium bicarbonate, sodium hydride, potassium hydride, and calcium hydride.

In a preferred embodiment, the organic base is selected from alkoxides, organic amines or any combination thereof, such as any one or a combination of any two or more of sodium methoxide, potassium methoxide, sodium ethoxide, potassium ethoxide, sodium tert-butoxide, potassium tert-butoxide, ethylamine, n-propylamine, isopropylamine, n-butylamine, ethylenediamine, dimethylethylenediamine, trimethylethylenediamine, tetramethylethylenediamine, trimethylamine, triethylamine, triethanolamine, DIPEA, pyridine, 4-dimethylaminopyridine, imidazole, methylimidazole, morpholine, N-methylmorpholine, piperidine, N-methylpiperidine, piperazine, N-methylpiperazine, tetrabutylammonium hydroxide, N,N-dimethylaniline, N,N-dimethylcyclohexylamine, hexamethylphosphoric triamide.

The salt of the compound of formula I is selected from salts formed with organic acids or inorganic acids, preferably pharmaceutically acceptable salts, such as hydrochloride, sulfate, phosphate, nitrate, acetate or benzoate.

In a preferred embodiment, the solvate of the compound of formula I is selected from solvates of a compound of formula I or a salt thereof with an organic solvent such as methanol, ethanol, isopropanol, diethyl ether, tetrahydrofuran, methyltetrahydrofuran, isopropyl ether, methyl tert-butyl ether, toluene, acetonitrile, acetone or butanone, preferably a solvate as described in the second aspect below.

In a more preferred embodiment, the method comprises one or more of the following steps:
(1) adding the compound of formula I or a salt or solvate thereof to a reaction solvent, and stirring to dissolve;
(2) adding inorganic base and organic base to purified water, stirring to dissolve, controlling the reaction temperature between -10 and 60 °C, adding the solution prepared in the step (1) dropwise into the aqueous solution of the base, and stirring until the reaction is completed; and
(3) concentrating the reaction mixture under reduced pressure to remove the reaction solvent, extracting with an organic solvent, adjusting the pH to 1-4 with an acid to precipitate the solid to obtain lifitegrast.

In a specific embodiment, the solid precipitated in step (3) is filtered, washed with water, and dried at 20-70 °C to obtain the product lifitegrast. The yield is ≥ 94.5%, the purity is ≥ 99.8%, the ee value is ≥ 99.8%, and the single impurity is ≤0.1%.

In step (1), the reaction solvent is selected from water, organic solvents or mixtures thereof, for example, any one or a combination of any two or more of methanol, ethanol, n-propanol, isopropanol, THF, methyltetrahydrofuran, diethyl ether, isopropyl ether, methyl tert-butyl ether, 1,4-dioxane, ethyl acetate, isopropyl acetate, isopropyl formate, toluene, xylene, acetonitrile, DMF, DMA, NMP, DMSO, acetone, butanone, n-hexane, cyclohexane, n-heptane, and purified water.

In step (2), the inorganic base and the organic base are as described above.

In step (3), the organic solvent used for extraction is selected from the group consisting of aprotic organic solvents, such as any one or a combination of any two or more of toluene, methyl formate, ethyl formate, isopropyl formate, methyl acetate, ethyl acetate, isopropyl acetate, n-hexane, cyclohexane, n-heptane, diethyl ether, isopropyl ether, methyl t-butyl ether, dichloromethane, and chloroform.

In step (3), the acid is selected from an organic acid or an inorganic acid, for example, any one of or a combination of any two or more of hydrochloric acid, hydrobromic acid, sulfuric acid, nitric acid, phosphoric acid, formic acid, acetic acid, benzoic acid.

In step (1), the salt of the compound of formula I is selected from salts formed with organic or inorganic acids, preferably pharmaceutically acceptable salts, such as hydrochloride, sulfate, phosphate, nitrate, acetate or benzoate. Said solvate is selected from solvates formed by the compound of formula I or salt thereof with an organic solvent such as methanol, ethanol, isopropanol, diethyl ether, tetrahydrofuran, methyltetrahydrofuran, isopropyl ether, methyl tert-butyl ether, toluene, acetonitrile, acetone, butanone. In a preferred embodiment, the solvate of the compound of formula I is a solvate as described in the second aspect below.

In a second aspect, the present invention provides a solvate of the compound of formula IV.

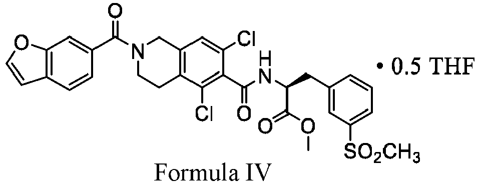

In a preferred embodiment, the compound of formula IV is in a crystalline form characterized by an X-ray powder diffraction pattern having characteristic diffraction peaks, expressed as the diffraction angle of 2θ, at 6.12 ± 0.2 °, 20.12 ± 0.2 ° and 20.94 ± 0.2 °.

In a more preferred embodiment, the compound of formula IV is in a crystalline form characterized by an X-ray powder diffraction pattern having characteristic diffraction peaks, expressed as the diffraction angle of 2θ, at 6.12 ± 0.2 °, 17.40 ± 0.2 °, 17.70 ± 0.2 °, 20.12 ± 0.2 °, 20.94 ± 0.2 ° and 22.5 ± 0.2 °.

In a more preferred embodiment, the compound of formula IV is in a crystalline form characterized by an X-ray powder diffraction pattern having characteristic diffraction peaks, expressed as the diffraction angle of 2Θ, at 6.12 ± 0.2 °, 17.40 ± 0.2 °, 17.70 ± 0.2 °, 20.12 ± 0.2 °, 20.94 ± 0.2 °, 22.5 ± 0.2 °, 22.78 ± 0.2 °, 26.14 ± 0.2 °, 26.74 ± 0.2 ° and 26.98 ± 0.2 °.

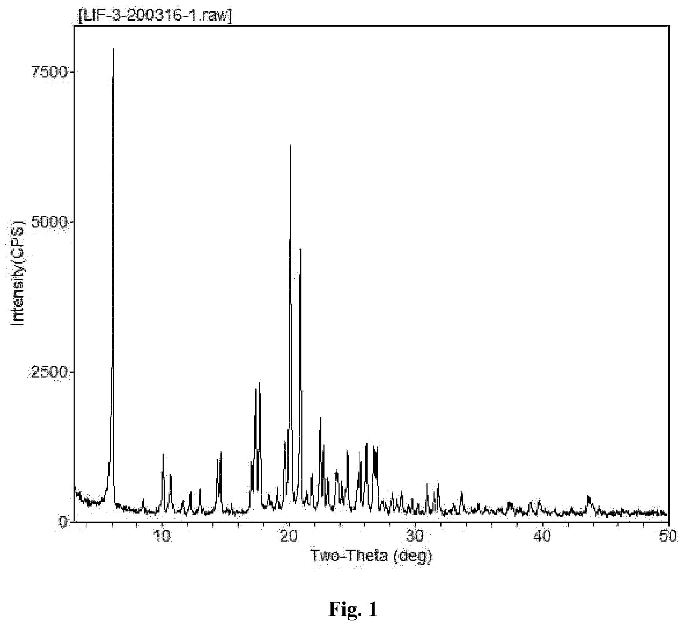

In a more preferred embodiment, the compound of formula IV is in a crystalline form characterized by an X-ray powder diffraction pattern as shown in Fig. 1.

The crystalline form of the compound of formula IV has an X-ray powder diffraction pattern as shown in Fig. 1 when Cu-kα (λ =1.54059 Å) radiation is used, the tube voltage is 40KV, the tube current is 250mA, the scanning speed is 5 °/min, the step width is 0.02 °, and the scanning range is 3-50 ° (20) from 0-20 continuous scanning. The main characteristic diffraction lines expressed by the 20 diffraction angle are shown in Table 1.

**Table 1: Main characteristic diffraction lines of the compound of formula IV**

| **Angle of diffraction 2θ (°)** | **Interplanar distance d (Å)** | **Relative Intensity I/I₀ (%)** |
|---|---|---|
| **6.12** | **14.43** | **100.0** |
| **10.10** | **8.76** | **12.6** |
| **10.680** | **8.28** | **8.1** |
| **12.26** | **7.22** | **4.6** |
| **13.00** | **6.81** | **5.1** |
| **14.38** | **6.15** | **11.6** |
| **14.64** | **6.05** | **13.1** |
| **17.08** | **5.19** | **10.6** |
| **17.40** | **5.09** | **26.0** |
| **17.70** | **5.01** | **27.3** |
| **19.70** | **4.50** | **13.4** |
| **20.12** | **4.41** | **78.3** |
| **20.94** | **4.24** | **55.3** |
| **21.84** | **4.06** | **7.0** |
| **22.50** | **3.95** | **19.9** |
| **22.78** | **3.90** | **13.9** |
| **23.08** | **3.85** | **6.6** |
| **23.76** | **3.74** | **7.8** |
| **23.90** | **3.72** | **7.6** |
| **24.18** | **3.68** | **6.1** |
| **24.66** | **3.61** | **11.9** |
| **25.46** | **3.50** | **6.4** |
| **25.64** | **3.47** | **12.5** |
| **26.14** | **3.40** | **14.2** |
| **26.74** | **3.33** | **13.5** |
| **26.98** | **3.30** | **13.5** |

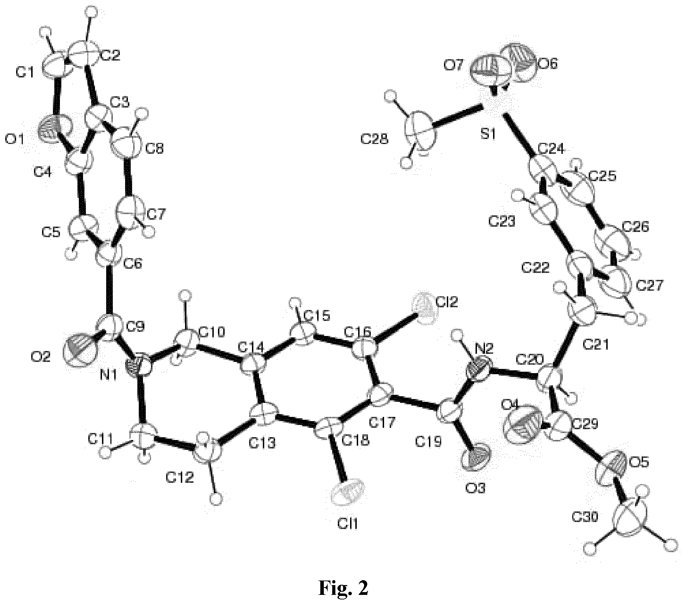

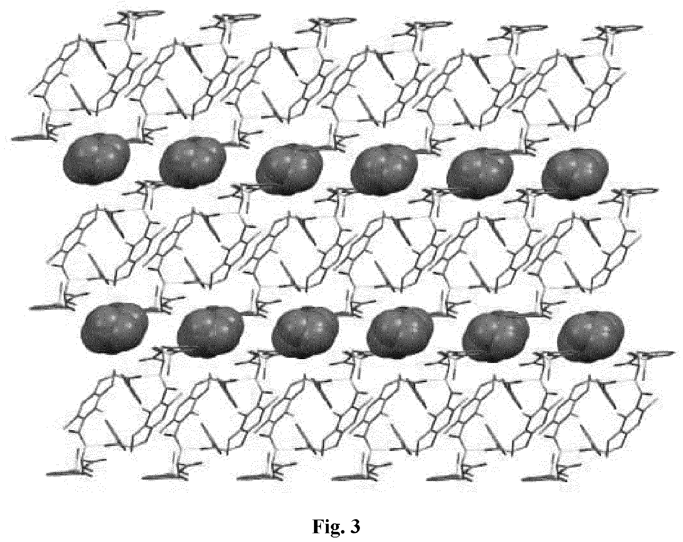

The single crystal structure data of the compound of formula IV of the present invention is shown in Table 2, the atomic coordinates and isotropic temperature factor of the molecules of the compound of formula IV are shown in Table 3, the three-dimensional structure diagram of the molecules of the compound of formula IV (solvent molecules omitted) is shown in Fig. 2, and the layered stacking (along the ac direction) of the molecules of the compound of formula IV is shown in Fig. 3.

The above results show that the crystal of the compound of formula IV belongs to the monoclinic crystal system, C2 (5#) space group, and the unit cell parameters are: a = 16.5592(7) Å, b = 13.3263(7) Å, ·c = 14.3904(7) Å, β = 97.039(2)°, V= 3151.6(3) Å³, Z=4, the molecular formula: C₃₀H₂₆Cl₂N₂O₇S0.5(C₄H₈O), crystal density d=1.403g/cm³. Its minimum asymmetric unit contains one molecule of the compound of formula IV (the structural formula is shown in Fig. 3) and half a tetrahydrofuran molecule, and there is no hydrogen bonding between the tetrahydrofuran molecule and the molecule of the compound of formula IV. The compound of formula IV connects two molecules of the compound of formula IV into a molecular dimer through intermolecular hydrogen bonding N-H...O, the molecular dimer is connected into a layered stacked structure through the weak interaction between the molecules, and THF molecules are positioned in the middle of the molecular layers of the compound of formula IV.

**Table 2: Single crystal structure data and structural correction data of molecule of the compound of formula IV**

| | |
|---|---|
| **Crystal shape, color, and size** | **Massive, colorless and transparent; 0.38mm×0.28mm×0.16mm** |
| **Crystal system** | **Monoclinic system** |
| **Space group** | ***C2*/*c*** |
| **Cell parameters** | ***a*=16.5592(7)Å, *b*=13.3263(7)Å, *c*= 14.3904(7)Å, *β* = 97.039(2)°** |
| **Cell volume** | **3151.6(3)Å³** |
| **Number of unit cell molecules** | **4** |
| **Molecular weight (Mr)** | **665.54** |
| **Density calculated (Dc)** | **1.403g/cm³** |
| **Absorption coefficient (µ)** | **0.325mm⁻¹** |
| **F (000)** | **1384** |
| **Final R factor** | **R₁=0.0308, wR₂=0.0807** |
| **Goodness-of -fit on F2 (S)** | **1.041** |
| **shift / su_max (δ/σ)** | **0.002** |
| **Residual error** | **0.450 e/ Å³(max), -0.313e/ Å³(min)** |
| **Flack** | **0.010(11)** |

| **Information on diffraction data acquisition and structural analysis:** | |
|---|---|
| **Type and wavelength of X-ray** | **CuKα, λ = 0.71073 Å** |
| **Total diffraction data/independent diffraction** | **61162 / 6979** |
| **I > 2sigma (I)** | **6610** |
| **Degree of data integrity** | **99.9 %** |
| **Number of modified variables/constraints** | **426/81** |
| **Structural correction method** | **Full matrix least squares correction based on F2** |
| **Temperature for diffraction data acquisition** | **170K** |

**Table 3: Atomic coordinates and isotropic temperature factor for the molecules of the compound of formula IV**

| **Atom(s)** | ***x*(*10⁴)** | ***y*(*10⁴)** | ***z*(*10⁴)** | **U(eq) (Å²*10³)** |
|---|---|---|---|---|
| **Cl1** | **6075.3(4)** | **1844.0(5)** | **5114.8(5)** | **38.79(15)** |
| **Cl2** | **8190.0(4)** | **4809.3(6)** | **6135.6(5)** | **42.51(17)** |
| **S1** | **7782.1(5)** | **7154.4(6)** | **8521.2(5)** | **46.09(19)** |
| **O1** | **4889.3(14)** | **8932.3(15)** | **2749.8(16)** | **45.6(5)** |
| **O2** | **3877.7(11)** | **4797.0(17)** | **2897.7(16)** | **45.8(5)** |
| **O3** | **7908.2(13)** | **2112.2(16)** | **6322.1(13)** | **43.4(5)** |
| **O4** | **6170.5(15)** | **1634.7(19)** | **8007.1(19)** | **58.3(6)** |
| **O5** | **7349.3(15)** | **883.3(17)** | **8537.0(16)** | **51.8(6)** |
| **O6** | **8397.9(18)** | **7881(2)** | **8825.6(19)** | **66.2(7)** |
| **O7** | **7026.4(16)** | **7206.7(19)** | **8916.2(17)** | **56.5(6)** |
| **N1** | **5235.5(12)** | **4837.0(17)** | **2833.6(15)** | **31.1(4)** |
| **N2** | **7147.7(14)** | **3069.5(17)** | **7183.2(15)** | **32.5(5)** |
| **C1** | **4641(2)** | **9692(2)** | **3299(2)** | **49.4(7)** |
| **C2** | **4297(2)** | **9346(2)** | **4026(2)** | **46.3(7)** |
| **C3** | **4308.6(17)** | **8266(2)** | **3964.5(19)** | **36.2(6)** |
| **C4** | **4674.9(16)** | **8060(2)** | **3163(2)** | **34.8(6)** |
| **C5** | **4804.6(15)** | **7103(2)** | **2836.5(18)** | **33.1(5)** |
| **C6** | **4527.3(15)** | **6322.0(19)** | **3348.2(18)** | **30.7(5)** |
| **C7** | **4153.6(17)** | **6515(2)** | **4156.9(19)** | **37.4(6)** |
| **C8** | **4048.1(18)** | **7471(2)** | **4475(2)** | **40.7(6)** |
| **C9** | **4532.0(15)** | **5257(2)** | **3014.1(18)** | **31.9(5)** |
| **C10** | **6042.7(15)** | **5199(2)** | **3202.3(18)** | **31.0(5)** |
| **C11** | **5241.1(17)** | **3771(2)** | **2576.8(19)** | **35.4(6)** |
| **C12** | **5402.8(16)** | **3151.2(19)** | **3468.0(19)** | **33.9(5)** |
| **C13** | **6105.7(15)** | **3569.2(18)** | **4124.5(17)** | **28.2(5)** |
| **C14** | **6421.9(15)** | **4526.3(18)** | **3991.9(17)** | **26.6(5)** |
| **C15** | **7076.8(14)** | **4897.5(19)** | **4591.6(17)** | **28.7(5)** |
| **C16** | **7394.1(15)** | **4334.7(19)** | **5359.4(18)** | **30.0(5)** |
| **C17** | **7082.5(16)** | **3399.6(19)** | **5539.9(17)** | **28.2(5)** |
| **C18** | **6451.0(15)** | **3031.0(18)** | **4904.4(18)** | **28.7(5)** |
| **C19** | **7422.4(16)** | **2796.0(19)** | **6385.4(18)** | **31.1(5)** |
| **C20** | **7428.7(18)** | **2561(2)** | **8051.6(19)** | **35.6(6)** |
| **C21** | **7407(2)** | **3270(2)** | **8889(2)** | **41.6(6)** |
| **C22** | **7971.7(18)** | **4158(2)** | **8853.6(18)** | **39.4(6)** |
| **C23** | **7666.7(18)** | **5124(2)** | **8724.0(18)** | **38.3(6)** |
| **C24** | **8196.5(19)** | **5942(3)** | **8708(2)** | **41.8(7)** |
| **C25** | **9035(2)** | **5807(3)** | **8811(2)** | **54.1(9)** |
| **C26** | **9335.1(19)** | **4842(4)** | **8941(3)** | **59.9(10)** |
| **C27** | **8817(2)** | **4028(3)** | **8964(2)** | **52.5(8)** |
| **C28** | **7556(2)** | **7220(3)** | **7297(2)** | **57.1(9)** |
| **C29** | **6896(2)** | **1653(2)** | **8181(2)** | **40.3(6)** |
| **C30** | **6917(3)** | **-11(3)** | **8744(3)** | **62.9(10)** |
| **O8** | **5219(6)** | **6467(7)** | **10536(7)** | **150(4)** |
| **C31** | **5779(8)** | **5754(12)** | **10366(14)** | **142(4)** |
| **C32** | **5404(11)** | **5010(13)** | **9795(16)** | **150(4)** |
| **C33** | **4553(10)** | **5109(12)** | **9910(16)** | **146(4)** |
| **C34** | **4444(9)** | **6111(13)** | **10113(16)** | **159(5)** |

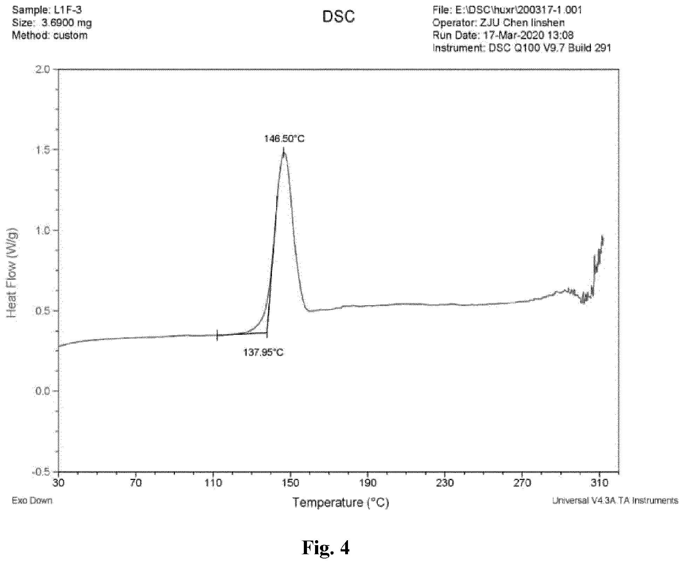

The DSC curve of the crystal of the compound of formula IV of the present invention is shown in Fig. 4, which shows that the melting point is 146.5 °C.

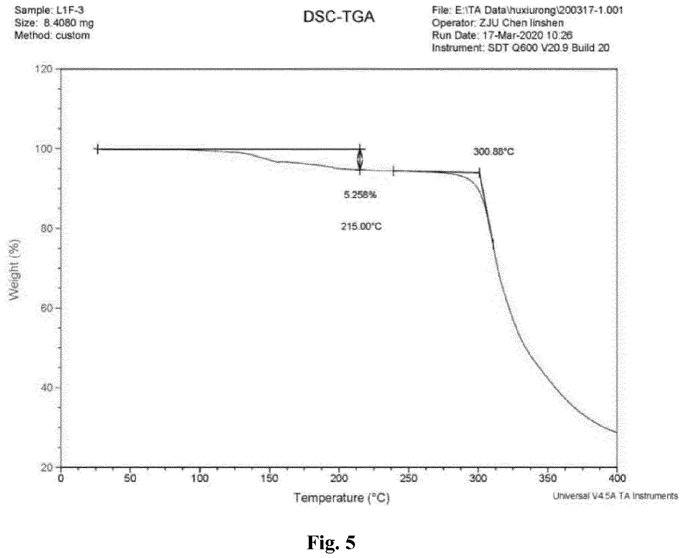

The DSC-TGA curve of the crystal of the compound of formula IV of the present invention is shown in Fig. 5, which indicates that the structure contains about 5.3% of solvent. This is consistent with single crystal diffraction analysis results. From the single crystal diffraction results, the cross-linked intermediate of the compound of formula IV is a THF solvate, i.e., the structure contains half a tetrahydrofuran molecule, and the molecular formula is: C₃₀H₂₆Cₗ₂N₂O₇S·0.5(C₄H₈O), molecular weight is 665.57. The weight ratio of half tetrahydrofuran is: 36.06/665.57*100% = 5.4% (the molecular weight of tetrahydrofuran is 72.11, and half tetrahydrofuran is 36.06).

In a third aspect, the present invention provides a method for the preparation of a solvate of formula IV, comprising:
(a) adding the crude product of the compound of formula III into a crystallization solvent, and after dissolving, cooling to precipitate crystal to obtain the compound of formula IV in a crystalline form,

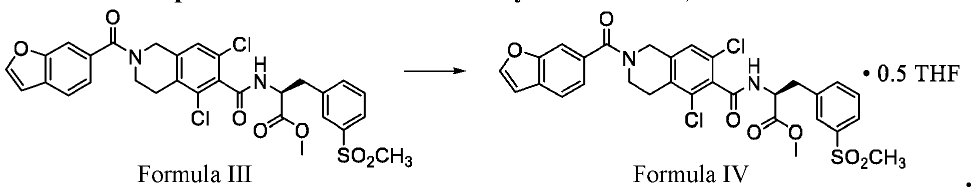

In one embodiment, step (a) is adding the crude compound of formula III to a crystallization solvent, heating to dissolve, then cooling to precipitate crystal, filtering and drying to obtain the compound of formula IV in a crystalline form.

The crystallization solvent is selected from: tetrahydrofuran, or a mixture of tetrahydrofuran and a solvent selected from any one or two or more of methanol, ethanol, propanol, isopropanol, toluene, xylene, chlorobenzene, acetonitrile, 2-methyltetrahydrofuran, 1,4-dioxane, dichloromethane, chloroform, ethyl acetate, isopropyl ether, methyl tert-butyl ether, hexane, n-hexane, cyclohexane, and n-heptane.

In one embodiment, the temperature for dissolving is from 20 to 100 ° C.

In one embodiment, the process of cooling and crystallizing is a programmed cooling and crystallizing process, for example, the temperature is lowered at a rate of 5 to 15°C per hour.

In one embodiment, when the temperature is lowered to -10 to 25 °C, the temperature is maintained for crystallization for 0.5-10 hrs, and then the mixture is filtered to obtain a white wet product, which is dried at a drying temperature of 10 to 70 °C to obtain the compound of formula IV in a crystalline form.

In a fourth aspect, the present invention provides a method for the preparation of lifitegrast, which comprises starting from a solvate of formula IV and hydrolyzing it under alkaline condition to obtain lifitegrast, 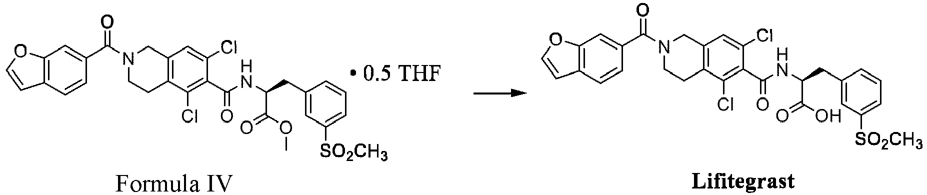 wherein the alkaline condition is that the hydrolysis is carried out in the presence of both an inorganic base and an organic base.

In a preferred embodiment, the inorganic base is selected from the group consisting of hydroxides, carbonates, bicarbonates, hydrides of alkali or alkaline earth metals or any combination thereof, for example, any one or a combination of any two or more of lithium hydroxide, sodium hydroxide, potassium hydroxide, calcium hydroxide, barium hydroxide, sodium carbonate, potassium carbonate, cesium carbonate, sodium bicarbonate, potassium bicarbonate, sodium hydride, potassium hydride and calcium hydride.

In a preferred embodiment, the organic base is selected from alkoxide, organic amine or any combination thereof, for example, any one or a combination of any two or more of sodium methoxide, potassium methoxide, sodium ethoxide, potassium ethoxide, sodium tert-butoxide, potassium tert-butoxide, ethylamine, n-propylamine, isopropylamine, n-butylamine, ethylenediamine, dimethylethylenediamine, trimethylethylenediamine, tetramethylethylenediamine, trimethylamine, triethylamine, triethanolamine, DIPEA, pyridine, 4-dimethylaminopyridine, imidazole, methylimidazole, morpholine, N-methylmorpholine, piperidine, N-methylpiperidine, piperazine, N-methylpiperazine, tetrabutylammonium hydroxide, N,N-dimethylaniline, N,N-dimethylcyclohexylamine, hexamethylphosphoric triamide.

In a more preferred embodiment, the method comprises one or more of the following steps:
(1') adding the crystal of the solvate of formula IV to a reaction solvent, and stirring to dissolve;
(2') adding inorganic base and organic base to purified water, stirring to dissolve, controlling the reaction temperature between -10 and 60 °C, adding the solution prepared in the step (1') dropwise into the aqueous solution of the base, and stirring until the reaction is completed;
(3') concentrating the reaction mixture under reduced pressure to remove the reaction solvent, extracting with an organic solvent, adjusting the pH to 1-4 with an acid to precipitate the solid to obtain lifitegrast.

In a preferred embodiment, the solid precipitated in step (3') is filtered, washed with water, and dried at 20-70 °C to obtain lifitegrast. The yield is ≥ 97.2%, the purity is ≥ 99.8%, the ee value is ≥ 99.8%, and the single impurity is ≤0.1%.

In a preferred embodiment, the reaction solvent is as described above for the first aspect.

In a preferred embodiment, the inorganic base and organic base in step (2') are as described above for the first aspect.

In a preferred embodiment, the extraction solvent is as described above for the first aspect.

In another embodiment, the method further comprises step (a) of the third aspect described above.

In a preferred embodiment, the method comprises:
(a) adding the crude product of the compound of formula III into a crystallization solvent, and after dissolving, cooling to precipitate crystal to obtain the solvate of formula IV in a crystalline form; and
(b) dissolving the crystal of the solvate of formula IV in a reaction solvent and hydrolyzing under alkaline condition to obtain lifitegrast, 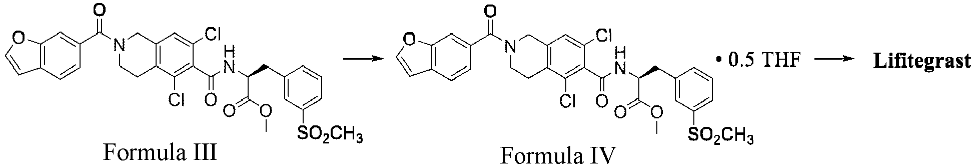
wherein the alkaline condition is that the hydrolysis is carried out in the presence of both an inorganic base and an organic base.

In a preferred embodiment, step (a) is as described above for the third aspect.

In a preferred embodiment, the reaction solvent of step (b) is selected from water, organic solvents or mixtures thereof, such as any one or a combination of any two or more of methanol, ethanol, n-propanol, isopropanol, THF, methyltetrahydrofuran, diethyl ether, isopropyl ether, methyl tert-butyl ether, 1,4-dioxane, ethyl acetate, isopropyl acetate, isopropyl formate, toluene, xylene, acetonitrile, DMF, DMA, NMP, DMSO, acetone, butanone, n-hexane, cyclohexane, n-heptane and purified water.

In a more preferred embodiment, said step (b) comprises steps (1'), (2') and (3') above.

The conditions of steps (1'), (2'), (3') are as described for steps (1), (2), and (3) in the corresponding embodiments of the first to third aspects above, respectively.

The present invention has unexpectedly discovered a method for preparing lifitegrast by hydrolyzing a compound of formula I or formula IV in the presence of an organic base and an inorganic base. Surprisingly, the method of the present invention has the advantages of high product purity, good ee value of the obtained product and few impurities, and overcomes the problems of low purity and low ee value of the product in the prior art. In particular, when the solvate of formula IV is used for preparing lifitegrast, the purity of the product can be greater than 99.9%, and the single impurity is less than 0.1% or even zero. Moreover, this method has a simple process, is very suitable for industrial production, and can significantly reduce costs.

### Drawings

Fig. 1 is an XRPD pattern of the crystal of the compound of formula IV.
Fig. 2 is the three-dimensional structure diagram of the crystal of the compound of formula IV (solvent molecules omitted).
Fig. 3 is the layered stacking diagram (along the ac direction) of the crystal of the compound of formula IV.
Fig. 4 is the DSC curve of the crystal of the compound of formula IV.
Fig. 5 is the DSC-TGA curve of the crystal of the compound of formula IV.

### Detailed Description

The method of the present invention is further illustrated by the following examples. It should be understood that the following examples are provided only for the purpose of enabling a better understanding of the present invention, and are not intended to limit the scope of the present invention in any way.

### Example 1: Method for the preparation of the compound of formula III

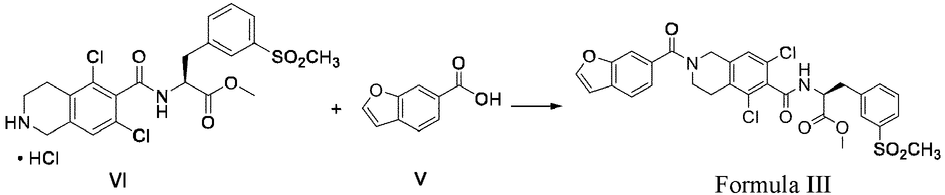

### Method 1:

Compound VI (46g), compound V (14.5g) and HATU (34.2g) were dissolved in 460g of DMF and the solution was cooled to -5 to 5 °C. Triethylamine (9.6g) was added dropwise, and after the dropwise addition was completed, the reaction was continued for 4-6 hours while keeping the reaction temperature. After the reaction was completed, the reaction solution was added dropwise to 500g of ice water and filtered. The filter cake was dissolved with 300g of dichloromethane and washed with water. The organic layer was collected and concentrated to dryness to give 55.5g of crude compound of formula III, yield = 100%, purity = 95.6%.

### Method 2:

Compound V (14.2g) and DMF (0.1g) were dissolved in 45g of dichloromethane and the mixture was heated to reflux, and 10.8g of thionyl chloride was added dropwise. After the reaction was completed, the reaction solution was concentrated to dryness, the residue was dissolved with 30g of dichloromethane and then added dropwise into a pre-prepared solution of 45g compound VI in 400g methylene chloride. After the reaction was completed, the mixture was washed twice with 100g of water, the layers were separated and the organic phase was concentrated to dryness to obtain 54.3g of crude compound of formula III, yield = 100%, purity = 95.5%.

### Example 2: Preparation of the compound of formula IV

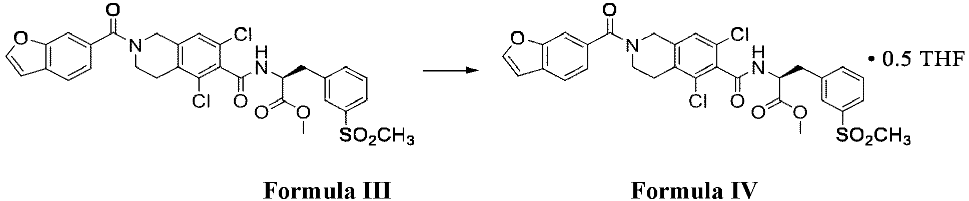

### Method 1:

The crude compound of formula III (20g) was weighed into a round-bottomed flask, 30g of tetrahydrofuran was added, the mixture was heated to reflux to dissolve, then 10g of methyl tert-butyl ether was added and the temperature was kept for 20 to 30 minutes. Then the mixture was cooled naturally to precipitate crystal and the stirring was continued for 1.0-1.5 hours at 5-10 °C. The mixture was filtered to obtain a white crystalline powder solid, which was oven-dried at 45-55 °C to obtain 18.9g of the product, yield = 94.5%, purity = 99.7%, and the maximum single impurity content was less than 0.1%.

### Method 2:

The crude compound of formula III (30g) was weighed into a reaction flask, 30g of tetrahydrofuran and 30g of isopropyl ether were added, the mixture was heated with stirring to obtain a solution, and stirring was continued for 10-20 minutes. The solution was subjected to programmed cooling to precipitate crystal, and the temperature was lowered at a rate of 5 to 10°C per hour. The temperature was kept at 30-40 °C for 1 hour. Then the temperature was continuously lowered under stirring to precipitate crystal. When the temperature reached 0-5°C, the mixture was continuously stirred for 1.0-1.5 hours. The crystal was filtered and oven-dried at 45-50°C to obtain 28.5g of white crystalline powder solid, yield = 95.0%, purity = 99.7%, and the maximum single impurity content was less than 0.1%.

### Method 3:

The crude compound of formula III (30g) was weighed into a reaction flask, 35g of tetrahydrofuran and 15g of n-heptane were added, the mixture was stirred and heated to reflux until completely dissolved, and stirring was continued for 10-20 minutes. The solution was subjected to programmed cooling to precipitate crystal, and the temperature was lowered at a rate of 5 to 15°C per hour. When the temperature was lowered to 0-10°C, the mixture was continuously stirred for 1.0-1.5 hours. Then the crystal was filtered and washed with n-heptane, oven-dried at 45-55°C to obtain 28.6g of white crystalline powder solid, yield = 95.3%, purity = 99.8%, and the maximum single impurity content was less than 0.1%.

### Example 3: Preparation of lifitegrast from the compound of formula II (i.e. compound of formula I wherein R = Bn)

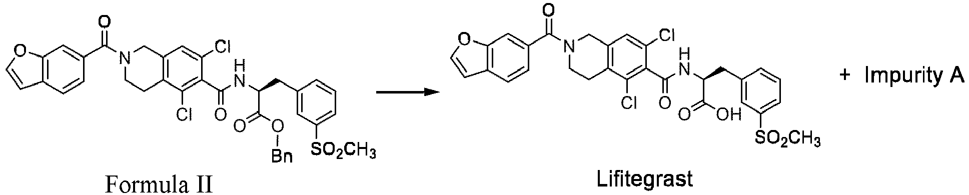

### Method 1 (inorganic base):

To 600g of acetonitrile was added 70.6g of the compound of formula II, the mixture was stirred to dissolve and the resulting solution was set aside for use. To 1000g of purified water was added 11.2g of potassium hydroxide, and the mixture was stirred to dissolve. Controlling the reaction temperature between -10 and 30 °C, and the reaction solution prepared above was added dropwise into the aqueous sodium hydroxide solution, and after the dropwise addition, the mixture was stirred until the reaction was completed. The reaction solvent was removed by concentration under reduced pressure, the concentrated solution was extracted with isopropyl acetate (2×200g), the pH was adjusted to 1-4 by addition of hydrochloric acid to precipitate a solid, which was filtered, washed with 1000g of water, and dried at 50-60 °C to obtain 55.6g of lifitegrast, yield = 90.4%, purity = 95.8%, ee value = 95.6%, and impurity A = 4.4%.

### Method 2 (inorganic base + organic base):

To 600g of acetonitrile was added 70.6g of the compound of formula II, the mixture was stirred to dissolve and the resulting solution was set aside for use. To 1000g of purified water were added 11.2g of potassium hydroxide and 15.0g of triethylamine, and the mixture was stirred to dissolve. Controlling the reaction temperature between -10 and 30 °C, and the reaction solution prepared above was added dropwise into the aqueous sodium hydroxide solution, and after the dropwise addition, the mixture was stirred until the reaction was completed. The reaction solvent was removed by concentration under reduced pressure, the concentrated solution was extracted with isopropyl acetate (2×200g), the pH was adjusted to 1-4 by addition of hydrochloric acid to precipitate a solid, which was filtered, washed with 1000g of water, and dried at 50-60 °C to obtain 58.3g of lifitegrast, yield = 94.8%, purity = 99.8%, ee value = 99.5%, and impurity A = 0.02%.

### Example 4: Preparation of lifitegrast from the compound of formula III (i.e. compound of formula I wherein R = Me)

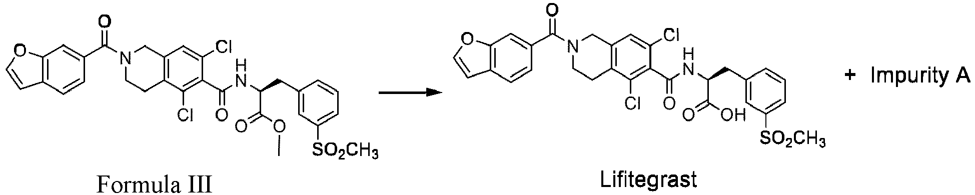

### Method 1 (inorganic base):

To 600g of acetone was added 63.0g of the compound of the formula III, the mixture was stirred to dissolve and the resulting solution was set aside for use.

To 1000g of purified water was added 10.0g of sodium hydroxide, and the mixture was stirred to dissolve. Controlling the reaction temperature to be -5 to 25 °C, and the reaction solution prepared above was added dropwise into the aqueous sodium hydroxide solution, and after the dropwise addition, the mixture was stirred until the reaction was completed. The reaction solvent was removed by concentration under reduced pressure, the concentrated solution was extracted with ethyl acetate (2×150g), the pH was adjusted to 1-4 by addition of hydrochloric acid to precipitate a solid, which was filtered, washed with 1000g of water, and dried at 55-65 °C to obtain 56.4g of lifitegrast, yield = 91.7%, purity = 96.2%, ee value = 94.9%, and impurity A = 5.2%.

### Method 2 (inorganic base + organic base):

To 600g of acetone was added 63.0g of the compound of the formula III, the mixture was stirred to dissolve and the resulting solution was set aside for use. To 1000g of purified water were added 10.0g of sodium hydroxide and 19.4g of DIPEA, and the mixture was stirred to dissolve. Controlling the reaction temperature to be -5 to 25 °C, and the reaction solution prepared above was added dropwise into the aqueous sodium hydroxide solution, and after the dropwise addition, the mixture was stirred until the reaction was completed. The reaction solvent was removed by concentration under reduced pressure, the concentrated solution was extracted with ethyl acetate (2×150g), the pH was adjusted to 1-4 by addition of hydrochloric acid to precipitate a solid, which was filtered, washed with 1000g of water, and dried at 55-65 °C to obtain 58.6g of lifitegrast, yield = 95.3%, purity = 99.8%, ee value = 99.9%, and impurity A = 0.01%.

### Example 5: Preparation of lifitegrast from the compound of formula IV

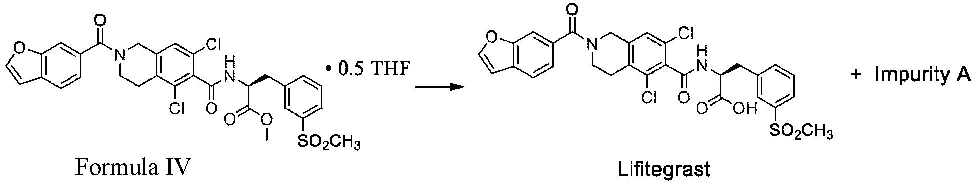

### Method 1 (inorganic base):

To 800g of tetrahydrofuran was added 66.6g of the compound of the formula IV, the mixture was stirred to dissolve and the resulting solution was set aside for use. To 1000g of purified water was added 10.0g of sodium hydroxide, and the mixture was stirred to dissolve. Controlling the reaction temperature to be -5 to 20 °C, and the reaction solution prepared above was added dropwise into the aqueous sodium hydroxide solution, and after the dropwise addition, the mixture was stirred until the reaction was completed. The reaction solvent was removed by concentration under reduced pressure, the concentrated solution was extracted with toluene (2×190g), the pH was adjusted to 1-4 by addition of hydrochloric acid to precipitate a solid, which was filtered, washed with 1100g of water, and dried at 50-70 °C to obtain 56.8g of lifitegrast, yield = 92.4%, purity = 96.5%, ee value = 96.7%, and impurity A = 3.3%.

### Method 2 (inorganic base + organic base):

To 800g of tetrahydrofuran was added 66.6g of the compound of the formula IV, the mixture was stirred to dissolve and the resulting solution was set aside for use. To 1000g of purified water were added 10.0g of sodium hydroxide and 15.5g N-methylpiperidine, and the mixture was stirred to dissolve. Controlling the reaction temperature to be -5 to 20 °C, and the reaction solution prepared above was added dropwise into the aqueous sodium hydroxide solution, and after the dropwise addition, the mixture was stirred until the reaction was completed. The reaction solvent was removed by concentration under reduced pressure, the concentrated solution was extracted with toluene (2×190g), the pH was adjusted to 1-4 by addition of hydrochloric acid to precipitate a solid, which was filtered, washed with 1100g of water, and dried at 50-70 °C to obtain 59.8g of lifitegrast, yield = 97.2%, purity = 99.9%, ee value = 100%, and impurity A = 0%.

## Claims

1. A method for preparing lifitegrast, which comprises:
hydrolyzing the compound of formula I or a salt or solvate thereof under alkaline condition to obtain lifitegrast, 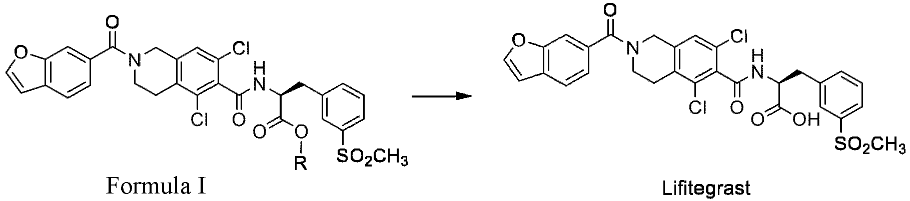
wherein R is selected from alkyl, haloalkyl, optionally substituted cycloalkyl, optionally substituted aryl or optionally substituted aralkyl, preferably C₁₋₁₀ alkyl, C₆₋₁₀ aryl, or C₆₋₁₀ aryl-C₁₋₁₀ alkyl, for example C₁₋₆ alkyl or C₆₋₁₀ aryl-C₁₋₆ alkyl, particularly preferably methyl, ethyl, n-propyl, isopropyl, n-butyl, isobutyl, tert-butyl or benzyl, and
the alkaline condition is that the hydrolysis is carried out in the presence of both an inorganic base and an organic base.

2. The method according to claim 1, wherein the inorganic base is selected from the group consisting of hydroxides, carbonates, bicarbonates, hydrides of alkali or alkaline earth metals or any combination thereof, for example, any one or a combination of any two or more of lithium hydroxide, sodium hydroxide, potassium hydroxide, calcium hydroxide, barium hydroxide, sodium carbonate, potassium carbonate, cesium carbonate, sodium bicarbonate, potassium bicarbonate, sodium hydride, potassium hydride, and calcium hydride.

3. The method according to any one of claims 1 to 2, wherein the organic base is selected from alkoxides, organic amines or any combination thereof, such as any one or a combination of any two or more of sodium methoxide, potassium methoxide, sodium ethoxide, potassium ethoxide, sodium tert-butoxide, potassium tert-butoxide, ethylamine, n-propylamine, isopropylamine, n-butylamine, ethylenediamine, dimethylethylenediamine, trimethylethylenediamine, tetramethylethylenediamine, trimethylamine, triethylamine, triethanolamine, DIPEA, pyridine, 4-dimethylaminopyridine, imidazole, methylimidazole, morpholine, N-methylmorpholine, piperidine, N-methylpiperidine, piperazine, N-methylpiperazine, tetrabutylammonium hydroxide, N,N-dimethylaniline, N,N-dimethylcyclohexylamine, hexamethylphosphoric triamide.

4. The method according to any one of claims 1 to 3, wherein the salt is selected from salts formed with organic acids or inorganic acids, preferably pharmaceutically acceptable salts, such as hydrochloride, sulfate, phosphate, nitrate, acetate, benzoate.

5. The method according to any one of claims 1 to 4, wherein the method comprises one or more of the following steps:
(1) adding the compound of formula I or a salt or solvate thereof to a reaction solvent, and stirring to dissolve;
(2) adding inorganic base and organic base to purified water, stirring to dissolve, controlling the reaction temperature between -10 and 60 °C, adding the solution prepared in the step (1) dropwise into the aqueous solution of the base, and stirring until the reaction is completed; and
(3) concentrating the reaction mixture under reduced pressure to remove the reaction solvent, extracting with an organic solvent, adjusting the pH to 1-4 with an acid to precipitate the solid to obtain lifitegrast.

6. A compound of formula IV

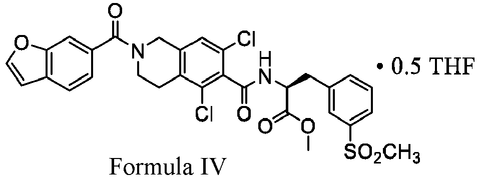

7. The compound according to claim 6, wherein the compound of formula IV is in a crystalline form **characterized by** an X-ray powder diffraction pattern having characteristic diffraction peaks, expressed as the diffraction angle of 2Θ, at 6.12 ± 0.2 °, 20.12 ± 0.2 ° and 20.94 ± 0.2.

8. The compound according to claim 6, wherein the compound of formula IV is in a crystalline form **characterized by** an X-ray powder diffraction pattern having characteristic diffraction peaks, expressed as the diffraction angle of 2θ, at 6.12 ± 0.2 °, 17.40 ± 0.2 °, 17.70 ± 0.2 °, 20.12 ± 0.2 °, 20.94 ± 0.2 ° and 22.5 ± 0.2 °.

9. A method for preparing a compound of formula IV according to claim 6, comprising:
(a) adding the crude product of the compound of formula III into a crystallization solvent, and after dissolving, cooling to precipitate crystal to obtain the compound of formula IV in a crystallization form,

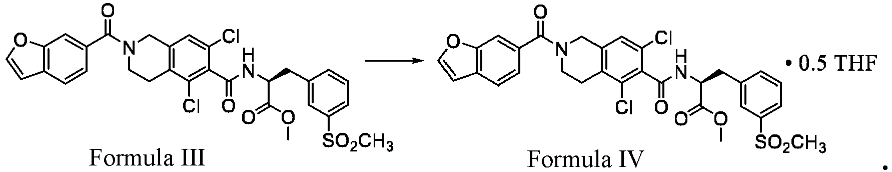

10. The method according to claim 9, wherein the crystallization solvent is selected from the group consisting of: tetrahydrofuran, or a mixture of tetrahydrofuran and a solvent selected from any one or two or more of methanol, ethanol, propanol, isopropanol, toluene, xylene, chlorobenzene, acetonitrile, 2-methyltetrahydrofuran, 1,4-dioxane, dichloromethane, chloroform, ethyl acetate, isopropyl ether, methyl tert-butyl ether, hexane, n-hexane, cyclohexane, and n-heptane.

11. A method for the preparation of lifitegrast, which comprises starting from the solvate of formula IV according to claim 6 and hydrolyzing it under alkaline condition to obtain lifitegrast, 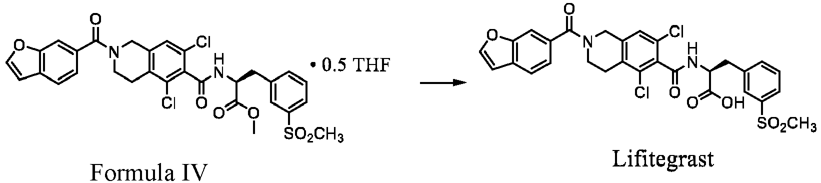 wherein the alkaline condition is that the hydrolysis is carried out in the presence of both an inorganic base and an organic base.

12. The method according to claim 11, wherein the inorganic base is as defined in claim 2.

13. The method according to any one of claims 11 to 12, wherein the organic base is as defined in claim 3.

14. The method according to any one of claims 11 to 13, wherein the method comprises one or more of the following steps:
(1') adding the crystal of the solvate of formula IV to a reaction solvent, and stirring to dissolve;
(2') adding inorganic base and organic base to purified water, stirring to dissolve, controlling the reaction temperature between -10 and 60 °C, adding the solution prepared in the step (1) into the aqueous solution of the base, and stirring until the reaction is completed;
(3') concentrating the reaction mixture under reduced pressure to remove the reaction solvent, extracting with an organic solvent, adjusting the pH to 1-4 with an acid to precipitate the solid to obtain lifitegrast.

15. The method according to any one of claims 11 to 14, wherein the method comprises:
(a) adding the crude product of the compound of formula III into a crystallization solvent, and after dissolving, cooling to precipitate crystal to obtain the solvate of formula IV in a crystallization form; and
(b) dissolving the crystal of the solvate of formula IV in a reaction solvent and hydrolyzing under alkaline condition to obtain lifitegrast, 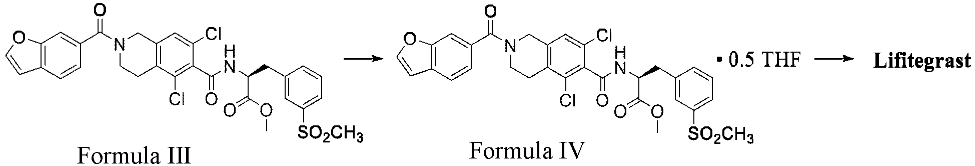
wherein the alkaline condition is that the hydrolysis is carried out in the presence of both an inorganic base and an organic base.

16. The method according to claim 15, wherein the crystallization solvent of step (a) is selected from the group consisting of: tetrahydrofuran, or a mixture of tetrahydrofuran and a solvent selected from any one or two or more of methanol, ethanol, propanol, isopropanol, toluene, xylene, chlorobenzene, acetonitrile, 2-methyltetrahydrofuran, 1,4-dioxane, dichloromethane, chloroform, ethyl acetate, isopropyl ether, methyl tert-butyl ether, hexane, n-hexane, cyclohexane, and n-heptane.

17. The method according to any one of claims 15 to 16, wherein the reaction solvent of step (b) is selected from water, organic solvents or mixtures thereof, such as any one or a combination of any two or more of methanol, ethanol, n-propanol, isopropanol, THF, methyltetrahydrofuran, diethyl ether, isopropyl ether, methyl tert-butyl ether, 1,4-dioxane, ethyl acetate, isopropyl acetate, isopropyl formate, toluene, xylene, acetonitrile, DMF, DMA, NMP, DMSO, acetone, butanone, n-hexane, cyclohexane, n-heptane and purified water.

18. The method according to any one of claims 15 to 17, wherein step (b) comprises steps (1'), (2') and (3') as defined in claim 14.

19. The method according to claim 5, wherein the reaction solvent is selected from water, organic solvents or mixtures thereof, such as any one or a combination of any two or more of methanol, ethanol, n-propanol, isopropanol, THF, methyltetrahydrofuran, diethyl ether, isopropyl ether, methyl tert-butyl ether, 1,4-dioxane, ethyl acetate, isopropyl acetate, isopropyl formate, toluene, xylene, acetonitrile, DMF, DMA, NMP, DMSO, acetone, butanone, n-hexane, cyclohexane, n-heptane and purified water.

20. The method according to any one of claims 5 and 14 to 18, wherein the organic solvent used for extraction is selected from the group consisting of aprotic organic solvents, such as any one or a combination of any two or more of toluene, methyl formate, ethyl formate, isopropyl formate, methyl acetate, ethyl acetate, isopropyl acetate, n-hexane, cyclohexane, n-heptane, diethyl ether, isopropyl ether, methyl tert-butyl ether, dichloromethane and chloroform.
